# EUROPEAN PATENT SPECIFICATION

(11) **EP 1 394 266 B1**
(45) Date of publication and mention of the grant of the patent: **14.11.2007**
(21) Application number: 02018056.8
(22) Date of filing: 12.08.2002
(51) Int. Cl.: C12Q 1/68

(54) **Detection of single nucleotide polymorphisms by single molecule analysis**
Nachweis von Einzelnukleotidpolymorphismen durch Einzelmolekülanalyse
Détection de polymorphismes mononucléotidiques par analyse des molécules individuelles

(43) Date of publication of application: 03.03.2004
(73) Proprietor: Gnothis Holding SA, 1015 Ecublens (CH)
(72) Inventor: Rigler, Rudolf, Prof.Dr., CH-1015 Lausanne (CH); Edman, Lars, Dr., 11256 Stockholm (SE)
(74) Representative: Weiss, Wolfgang

(56) References cited:
- WO-A-00/50172
- WO-A-02/38806
- DE-A- 10 058 915

## Description

The present invention relates to a method for determining a nucleotide polymorphism, particularly a single nucleotide polymorphism (SNP) in a nucleic acid target.

A method for determining nucleotide polymorphisms is described in WO02/38806. This method comprises the annealing of at least one starting primer to a nucleic acid template, wherein the 3'-end of the primer is located upstream of a nucleotide polymorphism to be analyzed, elongating the starting primer by incorporating at least one fluorescence-labelled nucleotide and detecting nucleotides incorporated into the starting primer by a single molecule analysis, particularly by subjecting the elongated starting primer to an exonuclease degradation and determining the sequence of liberated fluorescence-labelled nucleotides and oligonucleotides.

An object underlying the present invention was to provide a process for determining nucleotide polymorphisms, which can be performed in a simpler and faster manner as compared to the methods of the prior art.

This problem is solved by a method for determining a nucleotide polymorphism comprising:
(a) providing a nucleic acid template to be analyzed,
(b) annealing at least one primer to the nucleic acid template, wherein the 3'-end of the primer is located upstream of a nucleotide polymorphism to be analyzed,
(c) sequence-specific elongating the primer by incorporating at least one labelled nucleotide into the primer,
(d) degrading the nucleic acid template under conditions, wherein the elongated starting primer is substantially stable against degradation, and wherein the elongated primer is liberated from the nucleic acid template and
(e) detecting labels incorporated into the primer which are characteristic for the nucleotide polymorphism to be analyzed.

The method of the present invention allows a simple and accurate detection of nucleotide polymorphisms in nucleic acid target molecules. The method may be applied for diagnostic purposes, e.g. for determining the predisposition of diseases associated with specific nucleotide polymorphisms, e.g. hereditary diseases such as Huntington's disease, cystic fibrosis, Duchenne musculary dystrophy, but also specific forms of cancer such as breast cancer. Furthermore, the method of the invention is suitable for the determination or typing of microorganisms such as bacteria, protozoeae or viruses. Furthermore, the method allows the determination of nucleotide polymorphisms in the veterinary or agricultural field or for forensic applications. More particularly, the method of the invention allows the determination of several nucleotide polymorphisms on a single nucleic target in a single reaction.

The nucleotide polymorphism is preferably a single nucleotide polymorphism (SNP). The polymorphism, however, may comprise several nucleotides, e.g. two, three or more nucleotides. The nucleic acid template may comprise one nucleotide polymorphism as described above, preferably the nucleic acid template, however, comprises a plurality, e.g. at least two nucleotide polymorphisms. More preferably, the nucleic acid template comprises several nucleotide polymorphisms, which may be individually analyzed in a single reaction by using several primer molecules.

Step (a) of the method of the invention comprises providing a nucleic acid template, which may be e.g. DNA or RNA of any origin, as for instance DNA or RNA from viruses, prokaryotes, particularly pathogenic prokaryotes, Archeae or eukaryotes, particularly from mammals, more particularly from humans. However, one can also use recombinant DNA or RNA or synthetic DNA. The DNA or RNA is preferably employed in single-stranded form. Such single-stranded DNA can for instance be produced by reverse transcription of an RNA molecule by a reverse transcriptase, such as the reverse transcriptase of AMV (Avian myeloblastosis virus) or MMLV (Moloney murine leukemia virus). On the other hand, it is also possible to separate double-stranded DNA, such as genomic DNA, plasmid DNA or DNA of an episomal genetic element in order to obtain single-stranded DNA by means of heating, and, if necessary, to purify or to enrich one strand and subsequently anneal the primer.

The nucleic acid template may be contained in a complex sample, which optionally may be pre-purified to enrich the nucleic acid template, e.g. by specific hybridization to an immobilized capture probe, which is complementary to the nucleic acid template.

For the purpose of the present invention the nucleic acid template is preferably immobilized on a solid phase. More preferably, the solid phase is a particle, which may have an average size in the range of 0.5 to 10 *µ*m, especially 1 to 3 µm.

Examples of suitable materials of carrier particles are plastic materials, such as polystyrene, glass, quartz, metals and semimetals, such as silicon, metal oxides, such as silicon dioxide or compound materials containing several of the components mentioned above. Particularly preferred is the use of optically transparent carrier particles, such as for instance plastic materials or particles having a plastic core and a silicon dioxide shell.

The immobilization of the nucleic acid template on the carrier can be effected by covalent or non-covalent bonding, preferably via the 5'-end or via the 3'-end of the nucleic acid template. For example the immobilization on the carrier can be mediated via high-affinity interactions between the partners of a specific binding pair, such as biotin/streptavidin or avidin, hapten/anti-hapten-antibody, sugar/lectin etc.. For example, 5' or 3' biotinylated nucleic acid molecules can be coupled to streptavidin-coated carriers. Alternatively, the nucleic acid molecules can also be bound to the carrier adsorptively. For instance a binding of nucleic acid molecules carrying alkane thiol groups to metallic carriers, such as gold carriers, can be achieved. Still a further alternative consists in covalent immobilization, whereby the binding of the polynucleotides can be mediated via amino linkers to reactive silane groups on a silica-surface. Usually no more than one molecule of the template is bound to a single carrier particle. This can be easily achieved by means of a sufficiently high molar surplus of carrier particles as compared to template molecules.

Step (b) of the method of the invention comprises annealing of at least one primer to the nucleic acid template. The annealing is carried out under conditions which allow sequence-specific hydridization of the nucleic acid to a primer. The primer is an oligonucleotide or a nucleic acid analogue, comprising a sequence portion which is substantially complementary, preferably completely complementary to the nucleic acid template. The length of the complementary sequence portion is chosen such, that annealing under suitable assay conditions can take place. For example, the length of the complementary portion is at least 10, more preferably at least 12 nucleotides.

The primer, which is annealed to the nucleic acid molecule in step (b) is preferably stabilized against degradation in step (d). For this purpose, the primer may be a nucleic acid analogue comprising modified bonds between nucleotides and/or modified sugar groups, which result in an increased nuclease-resistance compared to the nucleic acid template molecule. For example, the primer may be a peptidic nucleic acid, wherein the phosphate sugar backbone is replaced by a peptidic backbone, e.g. consisting of 2-amino ethyleneglycin, which serves as a carrier for the nucleobases, e.g. A, T, G and C. It is, however, essential that the primer has a 3'-OH end function, which allows elongation according to step (c) of the invention.

The 3'-end of the primer is located upstream of the nucleotide polymorphism to be analyzed, preferably the 3'-end of the primer is located immediately (i.e. one nucleotide) upstream of the nucleotide polymorphism.

In step (c) of the present invention a sequence-specific elongation of the primer is carried out, which comprises the incorporation of at least one labelled nucleotide into the primer. The sequence-specific elongation is preferably effected by a template-dependent polymerase, e.g. by a DNA-dependent DNA polymerase such as T7 DNA polymerase or a thermostable DNA polymerase such as Taq, Pfu, Pwo and the like. More preferably, the polymerase does not exhibit substantial exonuclease activity under assay conditions. The labelling group is preferably a fluorescence group, e.g. a rhodamine, fluorescein or oxazine group as known in the art. Especially preferred are oxazine groups as described in DE 102 12 960.

Usually the sequence-specific elongation reaction is carried out in the presence of at least two, e.g. three or four different labelled nucleotides, preferably each carrying different labelling groups, in order to discriminate between different incorporated nucleotides. The discrimination between different labelling groups, e.g. fluorescence labelling groups may be carried out via the emission wavelength, the duration of the excited state or any combination thereof. In this manner a discrimination of four different labelling groups is possible.

The labelled nucleotide may be a desoxyribonucleotide, a ribonucleotide or another nucleotide which is accepted by the polymerase. In this case, the elongation reaction is preferably carried out under conditions wherein a limited number of nucleotides, e.g. 1-4 nucleotides are added to the primer. For this purpose, the concentration of polymerase and/or nucleotides may be kept sufficiently low. Preferably, however, the nucleotide is a chain termination molecule, i.e. the primer may be extended by only a single nucleotide. The chain termination molecule may be a didesoxynucleotide or any other suitable chain termination nucleotide.

The detection of the fluorescence can be carried out with any suitable measurement method, for instance by means of position- or/and time-resolved fluorescence-spectroscopy, which is able to detect fluorescence signals, even counting single photons, within a very small volume element, as is the case in a microchannel.

For example, the detection can be performed by means of confocal single molecule detection, such as fluorescence-correlation spectroscopy (FCS), whereby a very small, preferably confocal volume element, for instance 0.1 x 10⁻¹⁵ to 20 x 10⁻¹² I of the sample is exposed to the exciting light of a laser, exciting the fluorescence labels present in this measure volume to emit fluorescent light, the emitted fluorescence light of the measuring volume being measured by means of a photodetector, and a correlation between the time-related changes of the measured emission and the presence of a labelled molecule is established, so that single molecules in the measuring volume can be identified. With regard to the details of performing this process and details of the apparatus used in the detection process it is referred to the disclosure of European patent 0 679 251. The confocal single molecule determination has also been described by Rigler and Mets (Soc. Photo-Opt.lnstrum.Eng. 1921 (1993), 239 et seq.) and Mets and Rigler (J. Fluoresc. 4 (1994), 259-264).

Alternatively, or rather additionally, the detection can also be performed by means of a time-resolved decay measurement, a so-called time gating, such as described by Rigler et al., "Picosecond Single Photon Fluorescence Spectroscopy of Nucleic Acids", in: "Ultrafast Phenomena", D.H. Auston, Ed. Springer 1984. In this context the excitation of the fluorescence molecules is brought about within a measure volume and subsequently - preferably after a period of ≥ 100 ps - an opening of a detection interval at the photodetector. In this manner background signals created by Raman-effects can be kept at a sufficiently low level, in order to render possible an essentially undisturbed detection.

The detection of incorporated nucleotides preferably comprises a separation of the elongated primer from nucleotides that are not incorporated. This can be achieved by separating off non-incorporated nucleotides after the elongation step (c) by a bound-free separation or due to the differences in the migration velocity of nucleic acid molecules and non-incorporated nucleotides in an electrical field, as described for instance in the patent application DE 100 23 423. In this way it is usually possible to obtain an enrichment (or accumulation) by 10³ or more.

If the nucleic acid template is immobilised at a carrier particle, this particle can for example be trapped by means of an infrared laser. Subsequently, a washing step can take place in a directional flow, which can be electroosmotic or hydrodynamic. Due to the more favourable flow profile and the higher flow rates a hydrodynamic flow is preferred.

In a preferred embodiment the present invention allows a simultaneous determination and/or characterization of several polymorphisms on a single nucleic acid template molecule. For this purpose at least two primers are annealed to the nucleic acid template, wherein each primer is located upstream of a different nucleotide polymorphism and a time-resolved detection of individual primers is carried out. This may be achieved by a direction-specific degradation of the nucleic acid template molecule, wherein the primers are liberated at different times during the degradation procedure according to their location on the template. Alternatively, each primer may carry a specific label (distinguishable from the nucleotide labels) which allows correct identification. The degradation may be carried out, e.g. by enzymatic treatment, preferably by 5'→3' or 3'→5' exonuclease treatment. Suitable exonucleases are e.g. T7 DNA polymerase, T7 gene 6 exonuclease, E.coli exonuclease I, III and VII, bacteriophage lambda exonuclease, rec JF and trex 1,2.

In an especially preferred embodiment the detection is carried out as a single molecule detection, i.e. a single nucleic acid template molecule is analyzed. The single molecule detection preferably comprises the steps:
(i) introducing a particle having immobilized thereon a single molecule of the nucleic acid template into a detection apparatus comprising a microchannel,
(ii) trapping the particle at a predetermined position within the detection apparatus, and
(iii) degrading the nucleic acid template within the detection apparatus.

The detection and manipulation of loaded carrier particles can for instance be performed according to the methods described by Holm et al. (Analytical Methods and Instrumentation, Special Issue µTAS 96, 85-87), Eigen and Rigler (Proc.Natl.Acad.Sci. USA 91 (1994), 5740-5747) or Rigler (J.Biotech. 41 (1995), 177-186), which comprise a detection by means of a confocal microscope. The trapping of the loaded carrier particles in microchannel structures is preferably brought about by means of a capturing laser, i.e. an infrared laser. Suitable methods have for instance been described by Ashkin et al. (Nature 330 (1987), 24-31) and Chu (Science 253 (1991), 861-866).

The microchannels in the detection apparatus preferably have a diameter of 10 to 100 µm. The transport of a liquid through the detection apparatus can be brought about by electroosmotic or/and hydrodynamic flow. A transport by means of hydrodynamic flow in a parabolic flow profile is especially preferred.

In one embodiment of the invention the annealing of the primers to the nucleic acid template is performed prior to the introduction into the detection apparatus. The primer elongation can also be performed outside the detection unit, if necessary. In another embodiment the nucleic acid template is introduced into the detection apparatus before the annealing of the primers and the elongation of the primers take place.

The degradation of the nucleic acid templates takes place inside the detection apparatus. During the degradation procedure, the elongated labelled primers bound to the nucleic acid template are released and can be determined inside the apparatus. Preferably, direction-dependent degradation of the template is carried out resulting in a time-resolved release of primers according to their location on the template. The determination preferably comprises transport of the liberated labelled elongated primers to a detection zone within a microchannel wherein the type of label (which corresponds to a specific nucleotide polymorphism on the template) is determined. Preferably, the detection zone is a confocal volume element as described above.

## Claims

1. A method for determining a nucleotide polymorphism comprising:
(a) providing a nucleic acid template to be analyzed,
(b) annealing at least one primer to the nucleic acid template, wherein the 3'-end of the primer is located upstream of a nucleotide polymorphism to be analyzed,
(c) sequence-specific elongating the primer by incorporating at least one labelled nucleotide into the primer,
(d) degrading the nucleic acid template under conditions, wherein the elongated starting primer is substantially stable against degradation, and wherein the elongated primer is liberated from the nucleic acid template and
(e) detecting labels incorporated into the primer which are characteristic for the nucleotide polymorphism to be analyzed.

2. The method of claim 1, wherein the nucleic acid template is a single-stranded DNA or RNA molecule.

3. The method of claim 1 or 2, wherein the nucleic acid template is immobilized on a solid phase.

4. The method of claim 3, wherein the solid phase is a particle.

5. The method of claim 3 or 4, wherein the nucleic acid template is immobilized via its 5'-end or its 3'-end to the solid phase.

6. The method of any one of claims 1-5, wherein at least two primers are annealed to the nucleic acid template, wherein each primer is located upstream of a different nucleotide polymorphism to be analyzed.

7. The method of claim 6, wherein a time-resolved detection of individual primers is carried out.

8. The method of any one of claims 1-7, wherein the primer is stabilized against degradation.

9. The method of claim 8, wherein the primer is a nucleic acid analogue comprising modified bonds between nucleotides and/or modified sugar groups.

10. The method of any one of claims 1-9, wherein a single labelled chain termination nucleotide is incorporated into the primer.

11. The method of claim 10, wherein the chain-termination nucleotide is a didesoxynucleoside triphosphate.

12. The method of any one of claims 1-11, wherein the sequence-specific elongation is carried out in the presence of at least two different labelled nucleotides carrying different labelling groups.

13. The method of any one of claims 1-12, wherein the labelling groups are selected from fluorescence groups.

14. The method of any one of claims 1-13, wherein the degradation of the nucleic acid template molecule is direction-specific.

15. The method of any one of claims 1-14, wherein the nucleic acid template molecule is degraded by enzymatic treatment.

16. The method of claim 14 or 15, wherein the degradation is carried out by 5'→3' or 3'→5' exonuclease treatment.

17. The method of claim 16, wherein the exonuclease is selected from the group consisting of T7 DNA polymerase, T7 gene 6 exonuclease, E.coli exonuclease I, E.coli exonuclease III, E.coli exonuclease VII, bacteriophage lambda exonuclease, rec JF and trex 1,2.

18. The method of any one of claims 1-17, wherein at least two primers are liberated from the nucleic acid template sequentially and direction-specifically.

19. The method of any one of claims 1-18, wherein the detection is carried out as a single molecule detection.

20. The method of claim 19, wherein the single molecule detection comprises the steps:
(i) introducing a particle having immobilized thereon a single molecule of the nucleic acid template into a detection apparatus comprising a microchannel,
(ii) trapping the particle at a predetermined position within the detection apparatus, and
(iii) degrading the nucleic acid template within the detection apparatus.

## Patentansprüche

1. Verfahren zur Bestimmung eines Nukleotid-Polymorphismus umfassend:
(a) Bereitstellen einer zu untersuchenden Nukleinsäure-Matrize,
(b) Anlagern mindestens eines Primers an die Nukleinsäure-Matrize, wobei das 3'-Ende des Primers stromaufwärts von einem zu untersuchenden Nukleotid-Polymorphismus lokalisiert ist,
(c) Sequenz-spezifische Verlängerung des Primers durch Einbau mindestens eines markierten Nukleotids in den Primer,
(d) Abbauen der Nukleinsäure-Matrize unter Bedingungen, unter denen der verlängerte Start-Primer im Wesentlichen stabil gegenüber Abbau ist und unter denen der verlängerte Primer von der Nukleinsäure-Matrize freigesetzt wird und
(e) Nachweisen der Markierungen, die in den Primer eingebaut worden sind und die charakteristisch sind für den zu untersuchenden Nukleotid-Polymorphismus.

2. Verfahren nach Anspruch 1, wobei die Nukleinsäure-Matrize ein Einzelstrang DNA- oder RNA-Molekül ist.

3. Verfahren nach Anspruch 1 oder 2, wobei die Nukleinsäure-Matrize auf einer Festphase immobilisiert ist.

4. Verfahren nach Anspruch 3, wobei die Festphase ein Partikel ist.

5. Verfahren nach Anspruch 3 oder 4, wobei die Nukleinsäure-Matrize über dessen 5'-Ende oder dessen 3'-Ende an der Festphase immobilisiert ist.

6. Verfahren nach einem der Anprüche 1-5, wobei mindestens zwei Primer an die Nukleinsäure-Matrize angelagert werden und wobei jeweils ein Primer stromaufwärts von einem unterschiedlichen Nukleotid-Polymorphismus, der untersucht werden soll, lokalisiert ist.

7. Verfahren nach Anspruch 6, wobei ein zeitaufgelöster Nachweis der einzelnen Primer durchgeführt wird.

8. Verfahren nach einem der Ansprüche 1-7, wobei der Primer gegenüber Abbau stabilisiert ist.

9. Verfahren nach Anspruch 8, wobei der Primer ein Nukleinsäure-Analogon ist, das modifizierte Bindungen zwischen Nukleotiden und/oder modifizierte Zucker-Gruppen umfasst.

10. Verfahren nach einem der Ansprüche 1-9, wobei ein einzelnes markiertes Ketten-Terminations-Nukleotid in den Primer eingebaut wird.

11. Verfahren nach Anspruch 10, wobei das Ketten-Terminations-Nukleotid ein Didesoxynukleosidtriphosphat ist.

12. Verfahren nach einem der Ansprüche 1-11, wobei die Sequenz-spezifische Verlängerung in Gegenwart von mindestens zwei unterschiedlichen markierten Nukleotiden, die unterschiedliche Markierungsgruppen tragen, ausgeführt wird.

13. Verfahren nach einem der Ansprüche 1-12, wobei die Markierungsgruppen aus Fluoreszenz-Gruppen ausgewählt sind.

14. Verfahren nach einem der Ansprüche 1-13, wobei der Abbau des Nukleinsäure-Matrizen-Moleküls richtungsspezifisch ist.

15. Verfahren nach einem der Ansprüche 1-14, wobei das Nukleinsäure-Matrizen-Molekül durch enzymatische Behandlung abgebaut wird.

16. Verfahren nach Anspruch 14 oder 15, wobei der Abbau durch Behandlung mit 5'→3' oder 3'→5' Exonuklease erfolgt.

17. Verfahren nach Anspruch 16, wobei die Exonuklease ausgewählt ist aus der Gruppe, bestehend aus, T7 DNA Polymerase, T7 Gen 6 Exonuklease, E. coli Exonuklease I, E. coli Exonuklease III, E. coli Exonuklease VII, Bakteriophag Lambda Exonuklease, rec JF und Trex 1, 2.

18. Verfahren nach einem der Ansprüche 1-17, wobei mindestens zwei Primer nacheinander und richtungsspezifisch von der Nukleinsäure-Matrize freigesetzt werden.

19. Verfahren nach einem der Ansprüche 1-18, wobei der Nachweis als ein Einzelmolekül-Nachweis durchgeführt wird.

20. Verfahren nach Anspruch 19, wobei der Einzelmolekül-Nachweis die Schritte umfasst:
(i) Einbringen eines Partikels, auf dem ein einzelnes Molekül der Nukleinsäure-Matrize immobilisiert ist, in eine Nachweis-Vorrichtung, die einen Mikrokanal umfasst,
(ii) Festhalten des Partikels an einer vorbestimmten Stelle innerhalb der Nachweis-Vorrichtung, und
(iii) Abbauen der Nukleinsäure-Matrize innerhalb der Nachweis-Vorrichtiung.

## Revendications

1. Procédé pour déterminer un polymorphisme nucléotidique comprenant :
(a) la fourniture d'une matrice d'acide nucléique à analyser,
(b) l'hybridation d'au moins une amorce à la matrice d'acide nucléique, où l'extrémité 3' de l'amorce est située en amont d'un polymorphisme nucléotidique à analyser,
(c) l'élongation spécifique de séquence de l'amorce par incorporation d'au moins un nucléotide marqué dans l'amorce,
(d) la dégradation de la matrice d'acide nucléique dans des conditions dans lesquelles l'amorce de départ ayant subi une élongation est sensiblement stable à la dégradation, et où l'amorce ayant subi une élongation est libérée de la matrice d'acide nucléique et
(e) la détection de marqueurs incorporés dans l'amorce qui sont caractéristiques du polymorphisme nucléotidique à analyser.

2. Procédé selon la revendication 1 où la matrice d'acide nucléique est une molécule d'ADN ou d'ARN simple brin.

3. Procédé selon la revendication 1 ou 2 où la matrice d'acide nucléique est immobilisée sur une phase solide.

4. Procédé selon la revendication 3 où la phase solide est une particule.

5. Procédé selon la revendication 3 ou 4 où la matrice d'acide nucléique est immobilisée par le biais de son extrémité 5' ou de son extrémité 3' à la phase solide.

6. Procédé selon l'une quelconque des revendications 1-5 où au moins deux amorces sont hybridées à la matrice d'acide nucléique, où chaque amorce est située en amont d'un polymorphisme nucléotidique différent à analyser.

7. Procédé selon la revendication 6 où une détection à résolution dans le temps d'amorces individuelles est réalisée.

8. Procédé selon l'une quelconque des revendications 1-7 où l'amorce est stabilisée contre la dégradation.

9. Procédé selon la revendication 8 où l'amorce est un analogue d'acide nucléique comprenant des liaisons modifiées entre des nucléotides et/ou des groupes glucidiques modifiés.

10. Procédé selon l'une quelconque des revendications 1-9 où un nucléotide de terminaison de chaîne marqué unique est incorporé dans l'amorce.

11. Procédé selon la revendication 10 où le nucléotide de terminaison de chaîne est un didésoxynucléoside triphosphate.

12. Procédé selon l'une quelconque des revendications 1-11 où l'élongation spécifique de séquence est réalisée en présence d'au moins deux nucléotides marqués différents portant des groupes de marquage différents.

13. Procédé selon l'une quelconque des revendications 1-12 où les groupes de marquage sont choisis parmi les groupes fluorescents.

14. Procédé selon l'une quelconque des revendications 1-13 où la dégradation de la molécule de matrice d'acide nucléique est spécifique de direction.

15. Procédé selon l'une quelconque des revendications 1-14 où la molécule de matrice d'acide nucléique est dégradée par traitement enzymatique.

16. Procédé selon la revendication 14 ou 15 où la dégradation est réalisée par un traitement avec une exonucléase 5'→ 3' ou 3'→ 5'.

17. Procédé selon la revendication 16 où l'exonucléase est choisie dans le groupe consistant en l'ADN polymérase de T7, l'exonucléase du gène 6 de T7, l'exonucléase I de E. coli, l'exonucléase III de E. coli, l'exonucléase VII de E. coli, l'exonucléase de bactériophage lambda, rec JF et trex 1,2.

18. Procédé selon l'une quelconque des revendications 1-17 où au moins deux amorces sont libérées de la matrice d'acide nucléique successivement et de manière spécifique de direction.

19. Procédé selon l'une quelconque des revendications 1-18 où la détection est réalisée sous forme de détection d'une seule molécule.

20. Procédé selon la revendication 19 où la détection d'une seule molécule comprend les étapes :
(i) introduction d'une particule sur laquelle est immobilisée une seule molécule de la matrice d'acide nucléique dans un appareil de détection comprenant un microcanal,
(ii) piégeage de la particule à une position prédéterminée dans l'appareil de détection, et
(iii) dégradation de la matrice d'acide nucléique dans l'appareil de détection.
